# EUROPÄISCHE PATENTSCHRIFT

(11) **EP 0 447 468 B1**
(45) Veröffentlichungstag und Bekanntmachung des Hinweises auf die Patenterteilung: **24.05.1995**
(21) Anmeldenummer: 90900807.0
(22) Anmeldetag: 25.11.1989
(51) Int. Cl.: C12N 9/50, C12N 15/57, A61K 38/48

(54) **ANCROD-PROTEINE, IHRE HERSTELLUNG UND VERWENDUNG**
ANCROD PROTEINS, THEIR PRODUCTION AND USE
PROTEINES D'ANCROD, LEUR PRODUCTION ET UTILISATION

(30) Priorität: 10.12.1988 DE 3841736
(43) Veröffentlichungstag der Anmeldung: 25.09.1991
(73) Patentinhaber: BASF Aktiengesellschaft, 67063 Ludwigshafen (DE)
(72) Erfinder: BACH, Alfred, D-6802 Ladenburg (DE); STRUBE, Karl-Hermann, D-6720 Speyer (DE); KOERWER, Wolfgang, D-6718 Gruenstadt (DE)
(86) Internationale Anmeldenummer: EP8901427
(87) Internationale Veröffentlichungsnummer: WO9006362

(56) Entgegenhaltungen:
- EP-A- 0 323 722
- Biochem.J., Band. 131, 1973 M.W.C. Hatton: "Studies on the Coagulant Enzymefrom Agkistrodon rhodostoma Venom ", pp. 799-807
- The Journal of Biological Chemistry, Band. 262, Nr. 26, 1987 Walter Kisiel etal: "Characterization of a Protein C Activator from Agkistrodon contortrixcontortrix Venom ", pp. 12607-12613
- Biochemistry, Band. 28, 1989 Brad A. McMullen et al: "Primary Structure of aProtein C Activator from Agkistrodon contortrix contortrix Venom ", pp. 674-679

## Beschreibung

Die Vorliegende Erfindung betrifft Ancrod-Proteine, deren Herstellung und Verwendung zur Prophylaxe und Therapie von Krankheiten.

Ancrod ist ein Fibrinogen-spaltendes Enzym, welches aus dem Gift der malaiischen Grubenotter (Agkistrodon rhodostoma) gewonnen werden kann und antikoagulatorische Eigenschaften besitzt (Biochem, J. 131, 799, 1973).

Ancrod besitzt ein Molekulargewicht von etwa 38 000 Dalton und einen Kohlenhydratanteil von etwa 38 %.

Das Herstellungsverfahren für Ancrod ist aufwendig. Die äußerst giftige malaiische Grubenotter muß in Schlangenfarmen gezüchtet und von Hand gemolken werden bevor mit der biochemischen Äufarbeitung des Sekrets zur Isolierung von Ancrod begonnen werden kann. Die Anwendungsdauer des so hergestellten Ancrods ist begrenzt, da nach 6 bis 8 Wochen Resistenzerscheinungen auftreten können, die vermutlich auf die Bildung von Ancrod-neutralisierenden Antikörpern zurückzuführen sind. Vereinzelt kommt es auch zu hämorrhagischen Komplikationen.

Es wurden nun Ancrod-Proteine gefunden und rein hergestellt, welche hinsichtlich der therapeutischen Anwendung und der Herstellung dem bisher erhältlichen Ancrod überlegen sind.

Gegenstand der Erfindung sind reine glykosylierte, partiell glykosylierte oder nicht glykosylierte Polypeptide mit folgender Aminosäuresequenz:
1 VIGGDECNIN EHRFLVAVYE GTX¹WTFICGG VLIHPEWVIT AEHCARRRMN
51 LVFGMHRKSE KFDDEQERYP KKRYFIRCX²K TRTSWDEDIM LIRLNKPVX³N
101 SEHIAPLSLP SNPPIVGSDC RVMGWGSINR RIHVLSDEPR CANINLHX⁴FT
151 MCHGLFRKMP KKGRVLCAGD LRGRRDSCNS DSGGPLICNE ELHGIVARGP
201 NPCAQPNKPA LYTSVYDYRD WVNNVIAGX⁵A TCSP
worin X¹, X², X³, X⁴ und X⁵ Reste natürlicher α-Aminosäuren sind.

Darin bedeuten die einzelnen Buchstaben die Aminosäuren (vgl. Lubert Stryer, Biochemie, 1979, S. 12, S. R. Vieweg).

Die Aminosäurereste X¹, X², X³, X⁴ und X⁵, die gleich oder verschieden sein können, stellen N, Q, S, T, G, D, E, K, R, P, vorzugsweise jedoch N, Q, S und T und insbesondere N und Q dar.

Die Erfindung betrifft weiter DNA-Sequenzen, die für die oben genannten Proteine kodieren, sowie Vektoren, die diese DNA-Sequenzen enthalten.

Die erfindungsgemäßen Proteine lassen sich gentechnisch nach bekannten Methoden herstellen.

So kann man aus dem Drüsengewebe einer malaiischen Grubenotter (Agkistrodon rhodostoma) mRNA isolieren und in doppelsträngige cDNA übersetzen. Nach Einsetzen dieser cDNA in einen kommerziell erhältlichen Klonierungsvektor z. B. λ gt 10 wird eine cDNA-Bibliothek angelegt. Die dabei verwendeten Methoden sind beispielsweise in Maniatis et al., Molecular Cloning, CSH Press, (1982) nachzulesen. Auch das Screening solcher Genbanken mit radioaktiv markierten Oligonukleotidsonden ist inzwischen eine vielfach verwendete und beschriebene Methode. Nach diesem Verfahren kann ein cDNA-Klon, der Homologie zur Oligonukleotidsonde besitzt isoliert und charakterisiert werden. Dieses Verfahren ist in "DNA cloning Vol I, IRL Press, 1985, beschrieben.

Die so charakterisierte cDNA ist mit Hilfe von Restriktionsenzymen leicht zugänglich. Die dabei entstehenden Fragmente, ggf. in Verbindung mit chemisch synthetisierten Oligonukleotiden, Adaptoren oder Genfragmenten, können benutzt werden, um die für das Protein kodierende Sequenzen zu klonieren. Der Einbau der Genfragmente bzw. synthetischen DNA-Sequenzen in Klonierungsvektoren, z. B. die handelsüblichen Plasmide M13mp oder pkk-223-3, erfolgt in bekannter Weise. Auch können die Gene oder Genfragmente mit geeigneten chemisch synthetisierten oder aus Bakterien, Phagen, Eukaryontenzellen oder deren Viren isolierten Kontrollregionen versehen werden, die die Expression der Proteine ermöglichen.

Die Transformation bzw. Transfektion geeigneter Wirtsorganismen mit den so erhaltenen Hybridplasmiden ist ebenfalls bekannt und eingehend beschrieben (M. Wigler et al., Cell 16 (1979) 777-785; F.L. Graham and A.J. van der Eb, Virology 52 (1973) 456-467). Auch können die Hybridplasmide mit entsprechenden Signalsequenzen versehen werden, die die Sekretion der Polypeptide ins Medium erlauben.

Bei der Expression in Säugerzellen kann man Vektoren verwenden, die das zu exprimierende Gen, in diesem Fall die "Ancrod"-cDNA, unter die Kontrolle des Maus-Metallothionein- oder des viralen SV40-Promotors setzt (J. Page Martin, Gene, 37 (1985) 139-144). Notwendig für die Expression ist das Vorliegen des Methionin-Startcodons und der Leader-/Prosequenz des Gens für das Ancrod-Protein. Man isoliert dann Klone, die Kopien dieser Vektoren als Episome oder ins Genom integriert besitzen. Besonders vorteilhaft sind die Integration und Expression des Fremdgens auf der Basis des Rinderpapillom-Virus. In Verbindung mit prokaryontischen Sequenzen, die für die Replikation in Bakterienzellen und eine Antibiotika-Resistenz codieren, ist der Aufbau von "shuttle"-Vektoren möglich. Konstruktion und Vermehrung des Plasmids erfolgen zunächst in Bakterienzellen; anschließend erfolgt die Umsetzung in die Eukaryontenzellen, z.B. in die Maus-Fibroblastenzellinie c127.

Auch andere Zellsysteme, z.B. Hefe und andere Pilze, Insektenzellen sowie tierische und humane Zellen wie z.B. CHO-, COS, L-und 293 Zellen, können in Verbindung mit geeigneten Expressionsvektoren zur Expression der klonierten cDNA verwendet werden.

Diese eukaryontischen Expressionssysteme besitzen den Vorteil, daß sie in der Lage sind, ihre Produkte effektiv und meist in nativer Form zu sezernieren. Ferner besitzen sie die Fähigkeit, ihre Produkte posttranslational zu modifizieren.

So erhält Ancrod-Protein bei der Expression in Eukaryontenzellen noch Glykosidseitenketten. Diese Seitenketten fehlen bei den in Bakterien hergestellten Polypeptiden. Die Glykosidseitenketten können auch enzymatisch mit Hilfe entsprechender Glykosidasen vollständig oder partiell entfernt werden. Die meisten in Bakterien exprimierten eukaryontischen Proteine, fallen in der Zelle als denaturierte Einschlußkörper an und müssen proteinchemisch renaturiert werden. Ferner sind Bakterien oft nicht in der Lage, die Initiatoraminosäure Methionin vom fertigen Protein abzuspalten. Durch die Verwendung von Sekretionssystemen können diese Schwierigkeiten umgangen werden (Donald Oliver, Ann. Rev. Microbiol. 39, 1985, 615-48; John Ghrayeb et al. The EMBO Journal 3, 1984, 2437-2442.

Aufgrund der Degeneration des genetischen Codes ist es aber auch möglich, andere DNA-Sequenzen, z.B. chemisch synthetisierte Gene mit unterschiedlicher DNA-Sequenz für die Expression von Ancrod-Proteinen zu benutzen. Mit Hilfe des klonierten Gens können Varianten von Ancrod-Protein mit ähnlicher Wirkung hergestellt werden.

Die für die Varianten kodierenden DNAs sind durch Mutagenisierung (Insertion, Deletion, Punktmutation, Hybridbildung) aus dem ursprünglich klonierten Gen hervorgegangen.

Die Reinigung der erhaltenen Polypeptide erfolgt durch deren Abtrennung aus dem Kulturmedium durch Affinitäts- und Ionenaustausch-Chromatographie oder durch hydrophobe Chromatographie nach bekannten Verfahren.

Die beanspruchten Polypeptide werden in reiner Form, das heißt frei von hämorrhagischen Rückständen aus dem Giftdrüsensekret der Schlange, mit einem Reinheitsgrad von über 97 % erhalten.

Gegenstand der Erfindung sind auch Arzneimittel, die das erfindungsgemäß hergestellte Ancrod-Protein enthalten, ggf. in einem pharmazeutisch verträglichen Träger oder Bindemittel. Die Arzneimittel können auch Kombinationen des erfindungsgemäß hergestellten Ancrod-Proteins mit anderen pharmakologisch wirksamen Therapeutika enthalten, wie z.B. mit Thrombolytika (tPA, Streptokinase), Hirudin oder Thromboxanrezeptor-Antogonisten

Weitere Ausgestaltungen der Erfindung sind in den Beispielen näher beschrieben.

Für gentechnische Methoden sei dazu z.B. auf das Handbuch von Maniatis et al. "Molecular Cloning", Cold Spring Harbor Laboratory, 1982 oder "DNA cloning" Vol I - III IRI Press 1985 - 87 Herausgeber D.M. Glover hingewiesen.

Die erfindungsgemäßen Polypeptide eignen sich zur Behandlung von Glomerulonephritis, Herzinfarkt, non-ischämischem Schlaganfall, peripheren arteriellen Durchblutungsstörungen (insbesondere Atherosclerosis obliterans, Thrombangitis obliterans, diabetische Mikroangiopathie und Morbus Raynaud), instabiler Angina pectoris, tiefer Venenthrombose und anderen Thrombosen, Rethrombosierung nach thrombolytischer Therapie, Rethrombosierung nach gefäßchirurgischen Eingriffen wie bei der Implantation arterieller oder venöser Gefäßplastiken sowie zur Vermeidung von Thrombosen im extrakorporalen Kreislauf.

Die hier beschriebenen reinen Polypeptide führen nicht zu den bisweilen nach Gabe von aus Schlangen isoliertem Ancrod beobachteten Hämorrhagien und thromboembolischen Komplikationen. Darüberhinaus haben die Polypeptide, die sich in der Zuckerstruktur von dem aus Schlangengift isolierten Enzym unterscheiden, den Vorteil, daß sie wesentlich länger als das Präparat, welches aus Schlangengift gewonnen wird, verabreicht werden können, ohne daß Resistenzerscheinungen auftreten.

Das Ancrod-Protein wird in Form einer physiologisch verträglichen Lösung verwendet. Die Lösung enthält zweckmäßig ein Konservierungsmittel wie z.B. Chlorbutanol. Ancrod-Protein wird im allgemeinen subkutan injiziert. Die Behandlung kann stationär oder, falls die regelmäßige, zur Überwachung der Therapie erforderliche Kontrolle der Fibrinogenkonzentration sichergestellt ist, auch ambulant durchgeführt werden.

Die intravenöse Gabe von Ancrod ist möglich, sollte jedoch nur in Ausnahmefällen und unter stationärer Beobachtung erfolgen.

Ancrod-Protein ist grundsätzlich individuell zu dosieren. Maßgebend ist das Verhalten der Fibrinogenkonzentration im Plasma. Sie ist langsam auf 70-100 mg/100 ml Plasma zu senken. Während der gesamten Behandlungszeit ist die Fibrinogenkonzentration auf Werte innerhalb dieses Bereiches einzustellen.

Unter diesen Bedingungen sind u. a. die Fließeigenschaften des Blutes ausreichend verbessert, die PAI-1- und PGI₂-Serumkonzentration erniedrigt und die Konzentration von tPA im Serum erhöht.

### Beispiel 1

### Proteinchemische Analyse von Ancrod

### 1.0 Partielle Bestimmung der Aminosäuresequenz von Ancrod

Ausgangspunkt für die Klonierung von Ancrod-Protein war die Identifizierung von Aminosäuresequenzen des kommerziell erhältlichen Glykoproteins aus Agkistrodon rhodostroma. Methodisch wurde dazu wie folgt vorgegangen: Die Disulfidbrücken des Glykoproteins wurden zunächst reduziert und anschließend carboxymethyliert. Ein Teil des erhaltenen carboxymethylierten Glykoproteins wurde mit Trypsin fragmentiert und die resultierenden (Glyko)peptide durch "reversed phase" (r)HPLC aufgetrennt. Die Aminosäuresequenz einzelner Peptidfraktionen wurde dann durch Gasphasensequenzanalyse ermittelt. Von einem weiteren Teil des carboxymethylierten Ancrods wurde die N-terminale Aminosäuresequenz ebenfalls durch Gasphasensequenzanalyse bestimmt.

### 1.1 Reduktion und Carboxymethylierung von Ancrod

3,5 ml (Proteinkonzentration 3,56 mg/ml) Ancrod (Handelsname ARWIN), gelöst in 0,1 M Phosphat, 0,1 M NaCl, 0,3 % Trichlor-Tertiärbutylalkohol pH 6,7-7,0 wurden zunächst erschöpfend gegen einen 50 mM Ammoniumacetat-Puffer pH 7,0 dialysiert. Die so erhaltene umgepufferte Proteinlösung wurde lyophilisiert. Das Lyophilisat wurde in 6 ml Reduktionspuffer (6 M Guanidin, 0,2 M Tris/HCl, 1 mM EDTA, 130 mM DTT, 0,01 % TWEEN 80, pH 8,6) gelöst und über Nacht bei 37^{o}C inkubiert. Die Carboxymethylierung wurde dann durch Zugabe von 300 mg Jodacetamid gestartet. Nach 1 h Inkubation bei 0^{o}C im Dunkeln wurde durch die Zugabe von 300 »l β-Mercaptoethanol die Reaktion gestoppt und die Probe erschöpfend gegen Wasser dialysiert. Das so erhaltene carboxymethylierte Protein wurde in 0,5 ml Portionen lyophylisiert, nachdem zuvor 25 »l (entsprechen ca. 50 »g) zur Bestimmung der N-terminalen Sequenz (1.2) entnommen worden waren.

### 1.2 N-terminale Sequenzierung von Ancrod

Die nach Reduktion und Carboxymethylierung erhaltene (Glyko)protein-Lösung (25 »l, siehe 1.1) wurde zur Trockne eingeengt. Nach Aufnahme in 20 »l Ameisensäure und Verdünnen mit 10 »l H₂O wurde die (Glyko)proteinlösung auf einen mit POLYBREN beschichteten Glasfaserfilter aufpipettiert. Danach wurde die Sequenzierung am Gasphasensequenator (Applied Biosystems) gestartet. Die Detektion der freigesetzten Aminosäuren erfolgte "on line", nach Derivatisierung mit PITC (Phenylisothiocyanat) und rHPLC-Auftrennung, als PTH Aminosäuren (Phenylthiohydantoine). Eine nähere Beschreibung der Vorgehensweise ist der Bedienungsanleitung des Gasphasensequenators zu entnehmen.

### 1.3 Fragmentierung von carboxymethyliertem Ancrod

4 mg carboxymethyliertes Ancrod wurden in 500 »l 0,1 M Tris/HCl, 1 M Guanidin, pH 8,5 gelöst und 100 »l (200 »g) Trypsin-Lösung (Sequencegrade, Boehringer, Mannheim) zugegeben. Nach 16 h Inkubation bei 37^{o}C wurde die Reaktion durch Erhitzen des Ansatzes für 5 min auf 95^{o}C gestoppt. Die Probe wurde dann bis zur weiteren Verwendung bei -20^{o}C aufbewahrt.

### 1.4 Fraktionierung der tryptischen Peptide

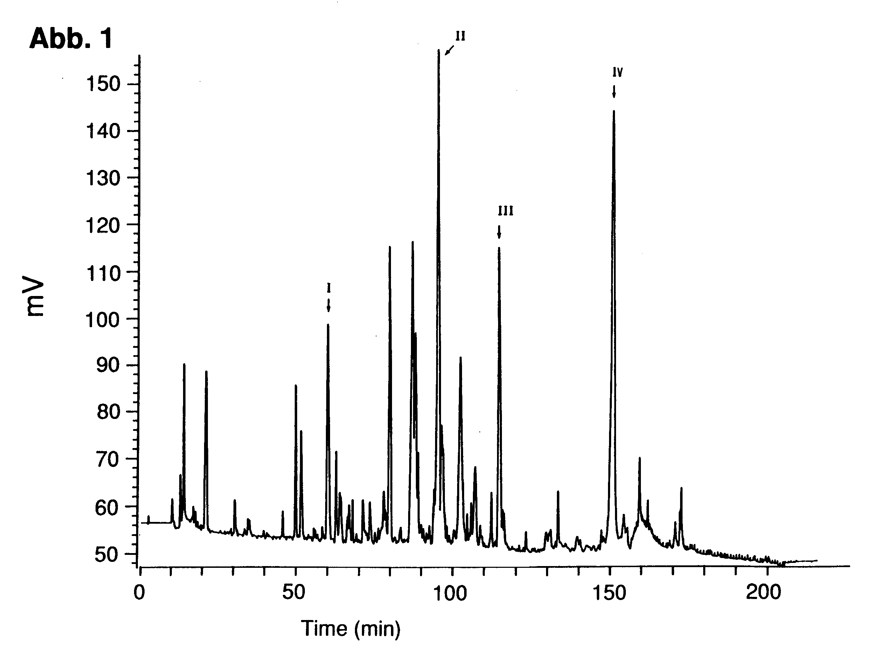

Das nach Reduktion, Carboxymethylierung und Trypsinverdau erhaltene (Glyko)peptidgemisch wurde durch rHPLC auf einer HP 1090 Anlage (Hewlett Packard) mit Hilfe einer VYDAK C-18 "widepore" Säule (Kat.-Nr. 201TP54) in einem TFA/Acetonitril Gradientensystem aufgetrennt. Ca. 1,3 mg Protein, entsprechend 200 »l aus 1.3, wurden auf die Säule appliziert und durch Anlegen eines linearen Acetonitrilgradienten (1-45 % in 190 min) eluiert. Der Verlauf der Chromatographie wurde durch Messung der UV-Absorption bei 230 nm verfolgt. Dabei wurde das in Abb. 1 gezeigte Chromatogramm erhalten. Die eluierten Peptide, in Abb. 1 mit I-IV bezeichnet, wurden in einem Konzentrator zur Trockne eingeengt. Die Aminosäuresequenz der so erhaltenen individuellen (Glyko)peptide wurde mit Hilfe des Gasphasensequenators ermittelt.

### 1.5 Identifizierte Aminosäuresequenzen

a) N-terminale Sequenzierung nach Reduktion und Carboxymethylierung
   V^{I}/_{K}GGDECN^{I}/_{K}NEHRFLVAVYEGT-WT
b) Aminosäure-Sequenzierung nach rHPLC-Trennung (zur Bezeichnung der Fraktionen siehe Abb. 1)
   I YFI/K
   II GPNPEAQPNKPALYTSIYDY
   III TSWDEDIMLIR
   IV FLVAVYEGT-WTFIEGGVLIHPEWVITAEH

### Beispiel 2

### Isolierung eines cDNA Klones für Ancrod aus der malaiischen Grubenotter (Agkistrodon rhodostoma)

1 g Giftdrüsengewebe einer 5 Jahre alten Schlange der Gattung Agkistrodon rhodostoma wurde in 6 M Guanidiniumthiocyanat, 5 mM Natriumcitrat (pH 7,0), 0,1 M 2-Mercaptoethanol, 0,5 % Sarcosyl im ULTRA-TURAX aufgeschlossen. Grobe Zelltrümmer wurden bei 3000 rpm abzentrifugiert. Die RNA wurde durch Zentrifugation durch ein 5,7 M CsCl-Kissen über Nacht bei 45.000 rpm abgetrennt. Anschließend wurde die polyA⁺-enthaltene RNA-Fraktion durch Affinitätschromatographie an oligo(dT)-Cellulose abgetrennt.

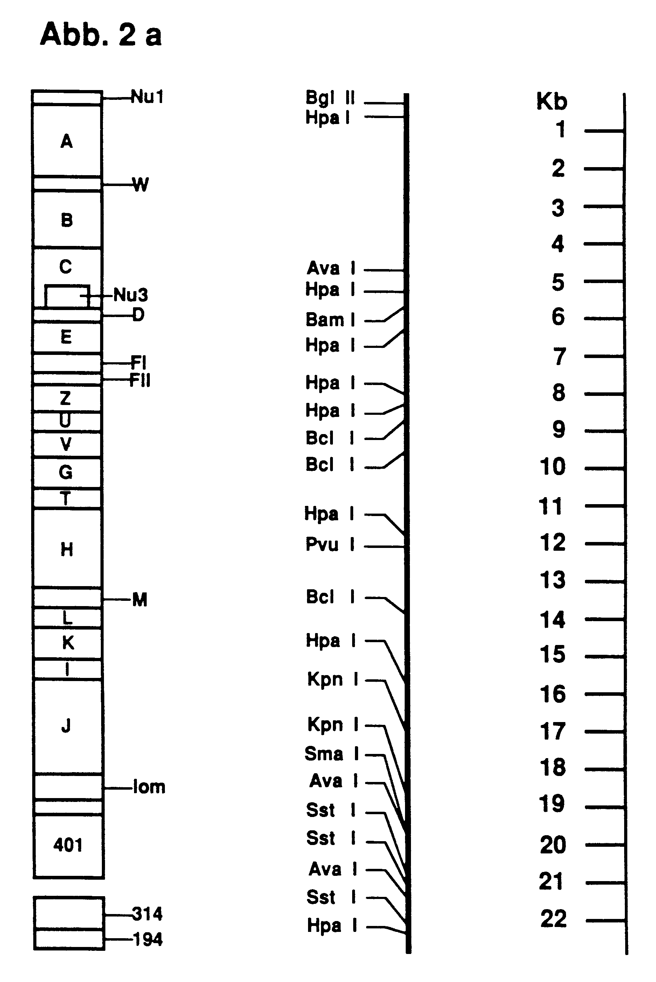

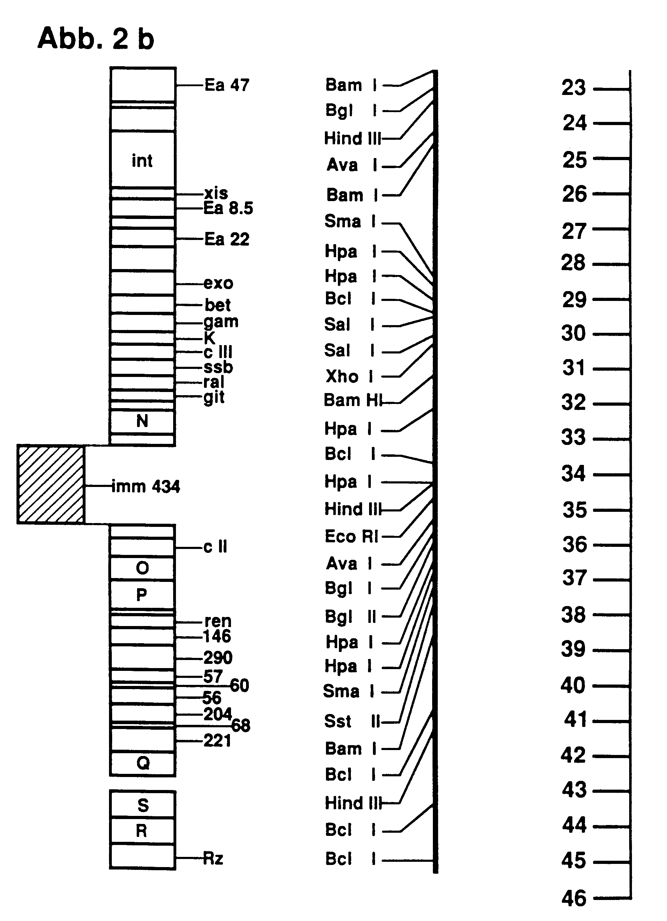

Mit Hilfe des Enzyms AMV-Reverse Transcriptase und oligo(dT)12-18 als Starter wurde die polyA⁺-RNA in einzelsträngige cDNA umgeschrieben. Die Synthese des zweiten Stranges erfolgte mit E.coli-DNA-Polymerase I. An die doppelsträngige cDNA wurde mit Hilfe des Enzyms T4-DNA-Ligase ein EcoRI Adaptor mit folgender Sequenz angesetzt: 5'AATT CCATGG ATG CATGC 3'. Der kommerziell erhältliche Phagenvektor λ gt 10 (Abb. 2) wurde mit dem Restriktionsenzym EcoRI linearisiert. Beide DNAs wurden miteinander ligiert und mit dem kommerziell erhältlichen Verpackungsextrakt zu infektiösen Phagen verpackt. Die rekombinanten Phagen wurden mit E. coli C 600 Hfl auf NZYDT-Platten ausplattiert und über Nacht bei 37^{o}C inkubiert. Die so erhaltene cDNA-Bibliothek enthielt 2x10⁶ unabhängige Klone. Nach Amplifikation der cDNA-Bibliothek entsprechend herkömmlicher Methoden wurden 500 000 Phagen mit C 600 Hfl Zellen ausplattiert. Die Phagen wurden auf Nitrocellulose-Filter übertragen, mit 0,5 N NaOH/1,5 M NaCl lysiert und die denaturierte DNA durch 2-stündiges Backen bei 80^{o}C fest an das Filter gebunden. Die Filter wurden in 6 x SET-Puffer (1 x SET = 0,15 M NaCl, 15 mM Tris/HCl, pH 7,4, 1 mM EDTA), 0,1 % SDS und 5 x Denhardt's Lösung (100 x Denhardt = 1 g Ficoll, 1 g Polyvinylpyrrolidon, 1 g BSA pro 50 ml) für 4 h, bei 68 ^{o}C vorhybridisiert. Mit Hilfe eines DNA-Synthesizers wurde eine Oligonukleotid-Sonde, ARNT, die 59 Basen umfaßt hergestellt. Sie besteht aus folgender Sequenz:
5' TGG ACT TTT ATT GAG GGC GGC GTG TTG ATT CAC CCG GAG TGG GTG ATT ACC GCC GAG CA 3'
Diese Sonde wurde am 5'-Ende mit γ-³²P-ATP markiert. Sie wurde dann mit den vorhybridisierten Filtern in einer Lösung, die 6 x SET, 0,1 % SDS, 30 % Formamid, 5 x Denhardt's und 10 % Dextransulfat enthielt, über Nacht bei 42^{o}C unter leichtem Schütteln inkubiert. Die Filter wurden danach mehrfach in 6 x SET/0,1 % SDS bei 42^{o}C gewaschen, angetrocknet und einem Röntgenfilm exponiert. Klone, die beim "Screening" eine radioaktive Antwort gaben, wurden isoliert und weitergezüchtet. Ein Klon, im folgenden AR4 genannt, enthielt ein etwa 0,9 kb großes Insert, das die codierende Region, sowie 5'- und 3'-nicht-kodierende Bereiche enthält. Phagen-DNA von AR4 wurde durch Inkubation der gereinigten Phagen mit Protenase K (ad 60 »g/ml) bei 55^{o}C für 1 h und anschließender Phenol/Chloroformextraktion präpariert. Nach Zugabe von 3 Volumen Ethanol (-20^{o}C) fiel die Phagen-DNA aus und wurde mit einer sterilen Injektionsnadel in 70 %igen Ethanol überführt, gewaschen und kurz sedimentiert. Nach kurzem Trocknen des Pellets an der Luft wurde es in TE-Puffer suspendiert.

### Beispiel 3

### Herstellung von einzelsträngiger DNA, die für Ancrod kodiert

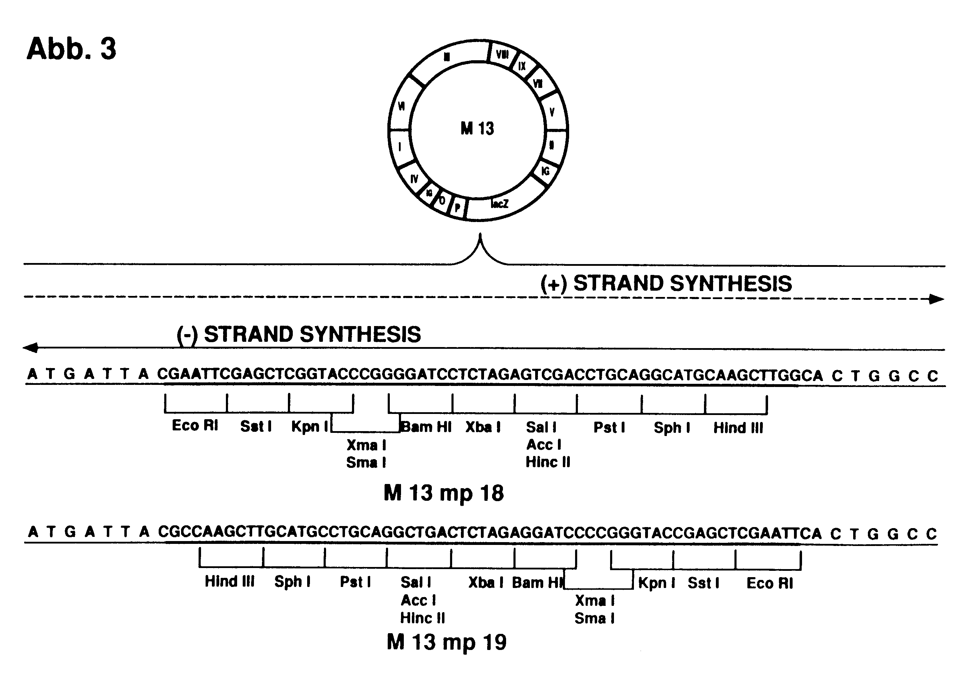

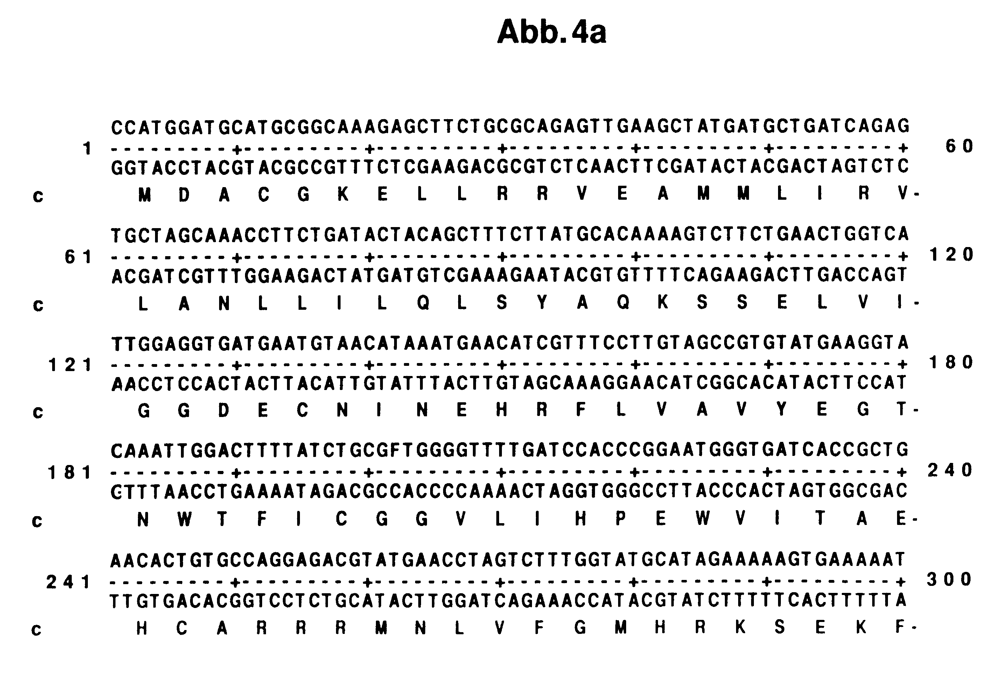

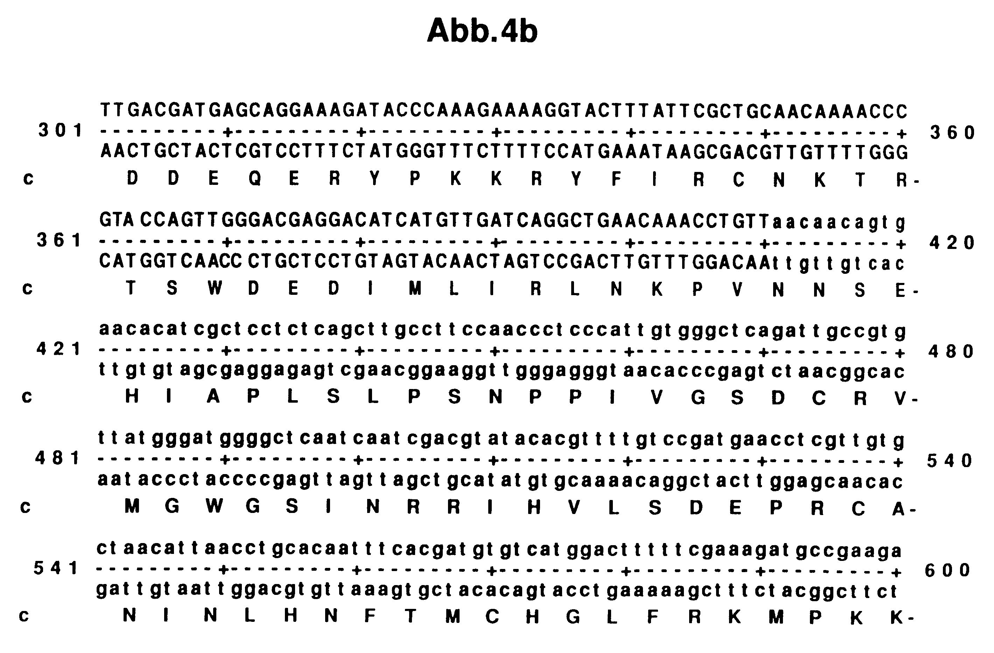

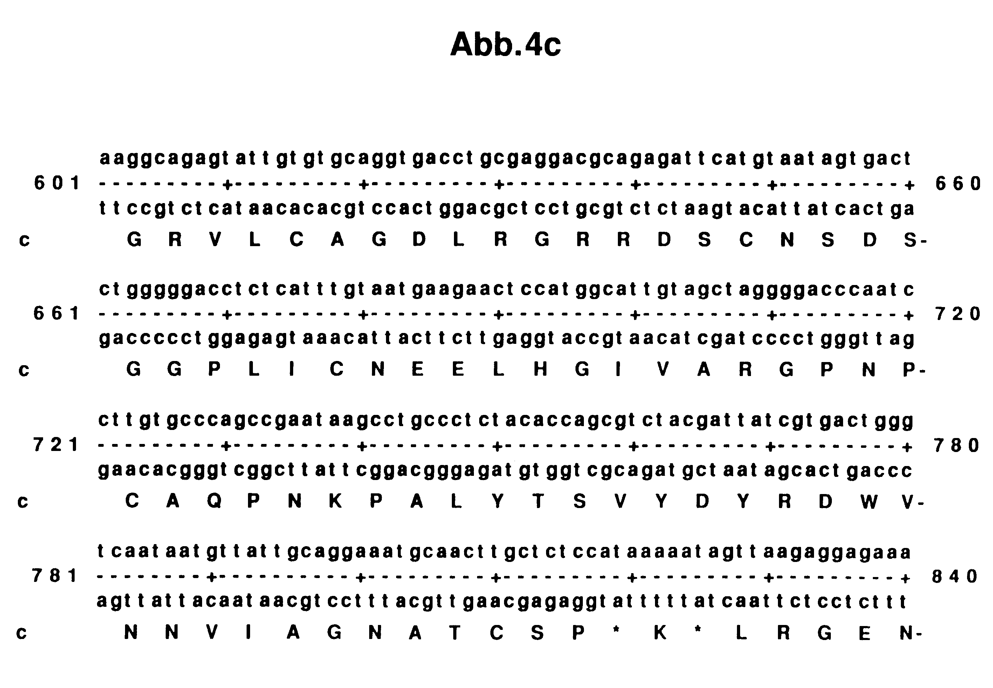

Ausgangspunkt war der in Beispiel 2 beschriebene Phagenklon AR4. Er wurde präparativ mit dem Restriktionsenzym Eco RI geschnitten. Das Eco RI-Fragment, das die Ancrod-kodierende DNA-Sequenz enthielt, wurde elektrophoretisch aus dem Gel eluiert. 30 ng dieses Fragmentes wurden bei 4^{o}C für 12 h mit 100 ng des Eco RI geschnittenen, kommerziell erhältlichen Klonierungsvektors M13mp18 oder M13mp19 (Abb. 3) ligiert. Das Volumen des Ligationsansatzes betrug 10 »l. Die Ligation wurde durch 5 min Erhitzen auf 80^{o}C beendet.

1/10 Volumen dieses Ligationsansatzes wurde zur Transformation von 100 »l kompetenten SR 101 Zellen eingesetzt. Nach Beendigung der Transformation wurden dem Transformationsansatz 60 »l 0,2 M IPTG-Lösung und 120 »l XGal (20 mg/ml) zugesetzt. Dieser Ansatz wurde in NZYDT-Topagar auf NZYDT-Agarplatten mit 200 »l SR 101 Zellen (OD₆₀₀=1) ausplattiert. Das Medium NZYDT ist kommerziell erhältlich (GIBCO-BRL). Klone, die die Ancrod-cDNA enthielten, konnten aufgrund fehlender Blaufärbung der Plaques identifiziert werden. Sie wurden als mpAR4 bezeichnet. Mittels DNA-Sequenzanalyse (Sanger et al., Proc. Natl. Acad. Sci. USA 74, 1977, 5463-67) wurde die DNA-Sequenz der Ancrod kodierenden cDNA aufgeklärt. (Abb. 4).

### 4.1 Konstruktion von Vektoren für die Expression von Ancrod in eukaryontischen Zellen

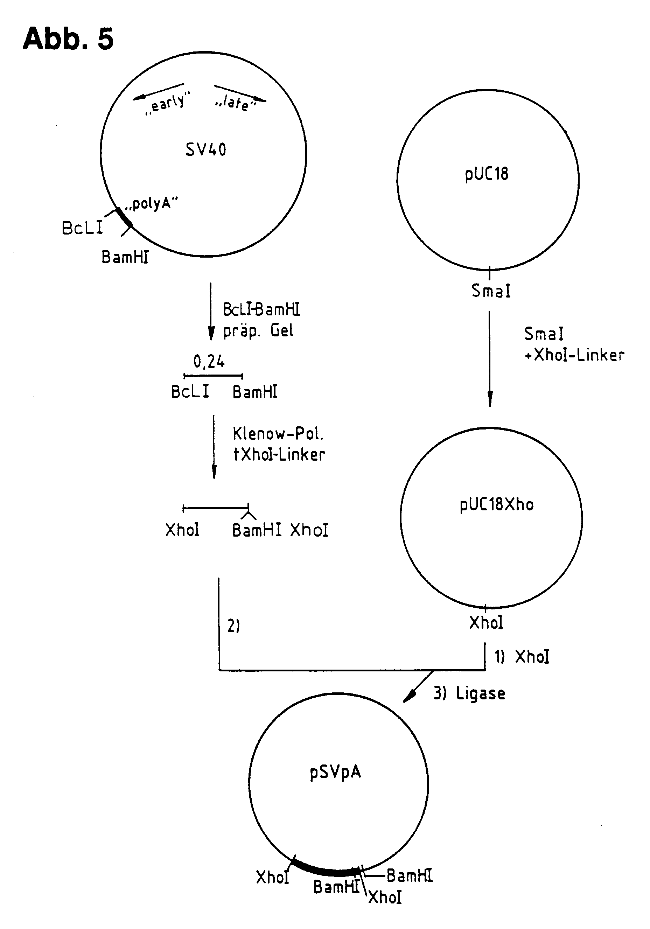

DNA des Affenvirus SV40 wurde mit den Restriktionsenzymen BamHI und BclI geschnitten und das 0,24 kb-Fragment gelelektrophoretisch präpariert (Abb. 5). Die Enden wurden in Gegenwart der vier Desoxynukleotidtriphosphate dATP, dCTP, dGTP und dTTP mit Klenow-Fragment aufgefüllt. Anschließend wurden XhoI-Linker anligiert.

Parallel wurde der kommerziell erhältlich Vektor pUC18 mit dem Enzym SmaI linearisiert. Dann wurden ebenfalls XhoI-Linker angesetzt. DNA dieses Vektors ("pUC18Xho") wurde mit XhoI linearisiert, mit alkalischer Phosphatase behandelt und mit dem 0,24 kb XholI-SV40-Fragment (s. o.) ligiert. Es entstand pSVpA.

pSVpA-DNA wurde präparativ mit XhoI gespalten und wie oben mit Klenow-Polymerase in Gegenwart der vier dNTPs inkubiert. Das 0,24 kb-Fragment wurde Gel-isoliert.

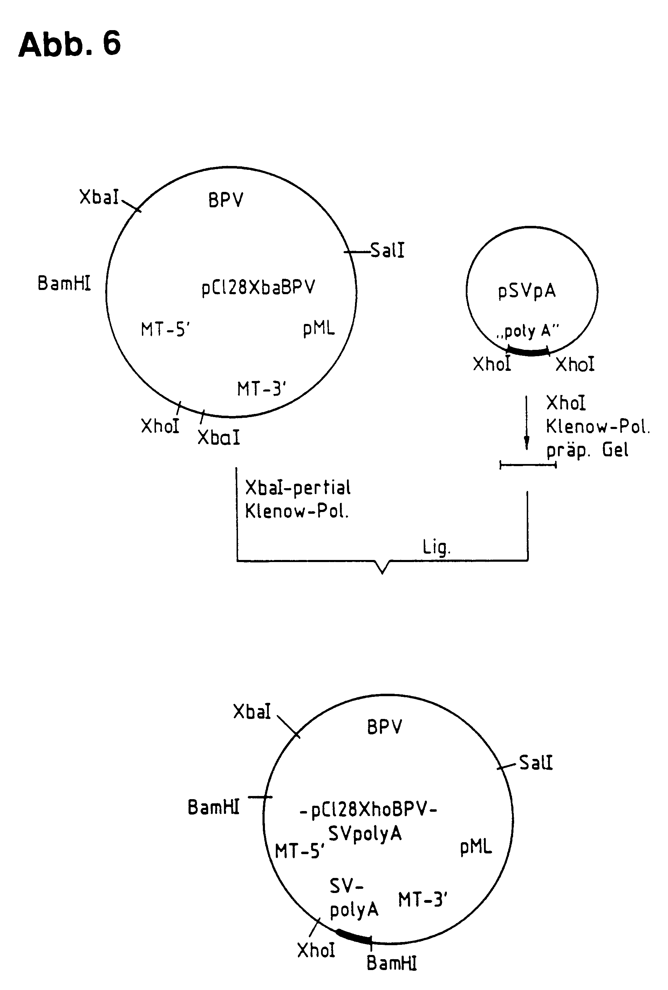

Gleichzeitig wurde der Eukaryonten-Expressionsvektor CL28XhoBPV, entstanden durch Ligation von CL28x und pB2-2 (nach Reddy et al. DNA 6, 1987, 461-72), partiell mit dem Restriktionsenzym XbaI geschnitten, d. h. es wurde zeitlich derart limitiert inkubiert, daß Moleküle entstanden, die nur an einer der beiden XbaI-Erkennungssequenzen gespalten, also linearisiert sind (Abb. 6). Der Ansatz wurde dann wie beschrieben mit Klenow-Polymerase und dNTPs umgesetzt. Die linearen Moleküle wurden anschließend durch Gelelektrophorese isoliert.

Es erfolgte dann die Ligation der linearen pCL28XhoBPV-Fragmente mit dem vorbehandelten 0,24 kb-Fragment aus SV40. Nach Transformation und Screening von Minilysaten wurde ein Klon isoliert, der das SV40-Fragment in der etwa 0,15 kB 3'-wärts der XhoI-Stelle gelegenen vormaligen XbaI-Stelle trug; diese DNA ("pCL28XhoBPV-SVpoly-A") trug die SV40-Transkriptionsstopsignale der "frühen" Gene.

Plasmid-DNA von pCL28XhoBPV-SVpolyA wurde mit dem Restriktionsenzym XhoI linearisiert und mit alkalischer Phosphatase behandelt. Gleichzeitig wurde mpAR4 mit dem Restriktionsenzym Eco RI gespalten und an das 0,9 Kb große Fragment Xho-Linker mittels T4 Ligase angehängt. Beide Fragmente wurden mittels T4-Ligase miteinander verbunden. Nach Transformation und Anlayse von Minilysaten wurde ein Klon isoliert, der die Ancrod-DNA einfach und in der korrekten Orientierung enthielt: pCL28BPV-Ancrod.

### Beispiel 5

### Transfektion und Etablierung von Zellinien

c127I Zellen (J. Virol. 26 (1978) 292; ATCC catalogue of cell lines and hybridomas 5th edition, 1985, p142) wurden mit BPV-Expressionsplasmiden transfiziert mit der Calciumphosphat-Copräzipitationsmethode (Virology 52 (1973)/456, DNA cloning; volume II, ed. D.M. Glover IRL Press, (1985) Seiten 143ff und 213).

5 x 10⁵ C127I-Zellen wurden in DMEM (Dulbeccos's Modified Eagles Medium) + 10 % FCS (Foetales Kalbsserum) in 60 mm Petrischalen eingesät. Am nächsten Tage wurde das Medium gewechselt auf MEM (Modified Eagles Medium) mit 25 nM Hepes + 10 % FCS. mit 10 ⁵g CsCl-gereinigter Plasmid DNA wurde ein Ca-Phosphat-Copräzipitat gebildet, welches vorsichtig auf die C127I-Zellen aufgebracht wurde. Die Zellen wurden 4 h bei 37^{o}C; 7 % CO₂ inkubiert. Durch eine anschließende Glycerin-Schock-Behandlung wurde die Effizienz der Transfektion erheblich gesteigert. Hierzu wurde 4 h nach Aufbringen des Präzipitates das Medium von den Zellen abgezogen. Die Zellen wurden 3 min mit je 2 ml 15 % Glycerin/HBS (DNA cloning Vol. II, Seite 152) in einer 60 mm Petrischale bei Raumtemperatur inkubiert. Die Glycerin/HBS-Lösung wurde abgezogen und der Zellrasen mit 3 ml DMEM + 10 % FCS gewaschen. Die Zellen wurden mit DMEM + 10 % FCS bei 37^{o}C; 7 % CO₂ inkubiert. Dreimal in der Woche wurde das DMEM + 10 % FCS abgezogen und durch frisches ersetzt. Nach 2 - 3 Wochen waren transfizierte Zellen, die das BPV-Genom enthalten, als Ansammlungen transformierter Zellen, sogenannte Foci, zu erkennen.

Nach Subklonierung der beschriebenen Foci wurden die Mediumüberstände der einzelnen Subklone nach bekannten Methoden auf Fibrinogen-spaltenden Aktivität untersucht.

Zur Produktion wurden die Zellinien nach Erreichen der Konfluenz in serumfreiem DMEM-Medium gehalten. Ancrod-Protein kann aus dem so erhaltenen serumfreien Zellkulturüberstand nach herkömmlichen proteinchemischen Methoden aufgereinigt und für pharmakologische und proteinchemische Analysen verwendet werden.

### Beispiel 6

### Herstellung von Ancrod-Protein in Bakterien

### 6.1 Mutagenisierung von mpAR4

2 pmol der einzelsträngigen mpAR4 wurde mit 5 pmol eines Oligonukleotids mit der Sequenz 5' CTC CAA TGA CCA TGG CAG AAG ACT TTT 3', die bis auf 3 Basenaustausche dem zu verändernden Bereich der mpAR4 DNA komplementär war, umgesetzt. Das Oligonukleotid wurde vorher mit Hilfe der T₄ Polynukleotidkinase phosphoryliert. Der Mutagenisierungsansatz sah wie folgt aus:

| | |
|---|---|
| einzelsträngige mpAR 4 | 1 »l (2 pmol) |
| kinasiertes Oligonukletid (inklusive ATP aus Kinasierungsreaktion) | 1 »l (5 pmol) |
| 10 x Ligase-Puffer | 5 »l |
| 1 mM dNTPs | 2,5 »l |
| 1 mM ATP | 4 »l |
| H₂O | 36,5 »l |

Der Mutagenisierungsansatz wurde 15 min bei 37^{o}C inkubiert, dann auf Raumtemperatur abgekühlt und schließlich mit 1 Einheit Klenow-Fragment (Boehringer Mannheim) versetzt. Nach 1 h Inkubation bei Raumtemperatur wurden 200 Einheiten T₄-Ligase (Biolabs) zugegeben. Der Ansatz wurde danach 16 h bei 4^{o}C inkubiert. Anschließend wurden 5 »l dieses Ansatzes zur Transformation von Komponenten SR 101 Zellen verwendet. Die daraus resultierenden Phagen-Plaques wurden auf Nitrozellulose übertragen. Danach wurden die Filter 5 min in 5xSSC gewaschen, an der Luft getrocknet und 2 h bei 80^{o}C "gebacken". Vorhybridisierung und Hybridisierung wurden wie in Beispiel 2 beschrieben durchgeführt. Das Waschen der Filter erfolgte mit 6xSET/0,1 % SDS bei einer Temperatur von 55-65^{o}C. Hybridisiert wurde mit dem Oligonukleotid, das in dem Mutagenisierungsansatz eingesetzt wurde. Positive Klone wurden autoradiographisch sichtbar gemacht. Positive Klone wichen in 3 Positionen von der mpAR4-Sequenz ab. Diese Substitution bewirkt, daß eine zusätzliche Erkennungsstelle für das Restriktionsenzym NcoI in der mpAR4 DNA vorhanden ist. Diese ermöglicht das Spalten dieser DNA vor dem Kodon (GTC), der ersten Aminosäure (Valin) des reifen Ancrod. Die so erhaltene DNA wurde mpAR4.1 genannt.

### 6.2 Subklonierung in pKK 233-2 und Expression in Bakterien

Durch limitierte Restriktion mit NcoI wurde aus der doppelsträngigen mpAR4.1 DNA ein Fragment freigesetzt, das eine Länge von ca. 720 Basenpaare aufweist. Das Fragment ist dadurch gekennzeichnet, daß es den gesamten kodierenden Bereich des reifen Ancrod-Proteins enthält. Dabei sieht der 5'-Bereich des kodierenden Strangs wie folgt aus:
5' C ATG GTC ATT GG....
Dieses NcoI Fragment wurde mit dem ebenfalls mit NcoI geschnittenen und kommerziell erhältlichen prokaryontischen Expressionsvektor pKK233-2 (CLONETECH Cat. 6003-1) ligiert. Das Ligationsgemisch wurde zur Transformation von E. coli JM 105 Zellen verwendet. Klone, die das rekombinante Plasmid enthielten, wurden durch die vielfach beschriebene Methode der Koloniehybridisierung identifiziert. Verwendet wurde dazu als Hybridisierungsprobe das in Beispiel 2 beschriebene Oligonukleotid ARNT. Die Hybridisierungs- und Waschbedingungen entsprachen ebenfalls den in Beispiel 2 beschriebenen Bedingungen. Klone, die mit der Probe hybridisierten, wurden mittels Restriktionsanalyse charakterisiert, um die Orientierung des integrierten Fragments zu bestimmen. Klone, die das Fragment in der zur Expression geeigneten Orientierung enthielten, wurden im entsprechenden Medium kultiviert. Bei einer OD₆₆₀ von 1 wurde IPTG zugesetzt, bis eine Endkonzentration von 10 mM erreicht wird. Nach weiterer Inkubation bei 37^{o}C für 4 h wurden die Zellen geerntet und das Polypeptid nach Standardverfahren gereinigt und renaturiert.

### Beispiel 7

### Herstellung von kohlenhydratfreiem Ancrod-Protein und partiell kohlenhydratfreier Ancrod-Proteine in Säugerzellen

### 7.0 Mutagenisierung des einzelsträngigen DNA

Das Plasmid mpAR4 wurde derart mutagenisiert, daß eine, einige oder alle der Kodon-Kombinationen für das Tripeptid NXT bzw. das Tripeptid NXS durch eine Kodonkombination für das Tripeptid QXT bzw. QXS ersetzt wurden. Die Mutagenisierung der einzelsträngigen DNA wurde wie in Beispiel 6.1 durchgeführt.

### 7.1 Einzelstrang-DNA des Klons mpAR4 wurde mit dem Nukleotid KH1 mutagenisiert.

KH1: 5' AAAAGTCCACTGTGTACCTTCAT 3'
Die mutierten Klone wurden mittels Plaque-Hybridisierung mit der Probe KH1 identifiziert. Hybridisiert wurde bei 37^{o}C in 20 % FA (Formamid) und 4xSSC für 16 h. Gewaschen wurde bei 60^{o}C in 5xSSC/0,1 SDS. Die resultierenden Klone wurden als mpAR4.KH1 bezeichnet.

### 7.2 Einzelstrang-DNA des Klons mp AR4.KH1 wurde mit dem Oligonukleotid KH₂ mutagenisiert.

KH2: 5 TAC GGGTTTTCTGGCAGCGAATAAA 3'
Die mutierten Klone wurden mittels Plaque-Hybridisierung mit der Probe KH2 identifiziert. Hybridisiert wurde bei 37^{o}C in 20 % FA und 5 x SSC für 16 h, gewaschen wurde bei 65^{o}C in 5xSSC/0,1 % SDS. Die resultierenden Klone wurden als mpAr4.KH1,2 bestimmt.

### 7.3 Einzelstrang-DNA des Klons mpAR4.KH1,2 wurde mit dem Oligonukleotid KH3 mutagenisiert.

KH3: 5' GTTCACTGTTCTGAACAGGTTTG 3'
Die mutierten Klone wurden mittels Plaque-Hybridierung mit der Probe KH3 identifiziert. Hybridisiert wurde bei 35^{o}C in 5xSSC für 16 h, gewaschen wurde bei 58^{o}C in 5xSSC/0,1 % SDS. Die resultierenden Klone wurden als mpAR4.KH 1,2,3 bezeichnet.

### 7.4 Einzelstrang-DNA desKlons mpAR44.KH1,2,3 wurde mit dem Oligonukletid KH4 mutagenisiert.

KH4: 5' ACATCGTGAACTGGTGCAGGTTAA
Die mutierten Klone wurden mittels Plaque-Hybridierung mit der Probe KH4 identifiziert. Hybridisiert wurde bei 37^{o}C in 20 % FA und 5xSSC für 16 h, gewaschen wurde bei 60^{o}C in 5xSSC/0,1 % SDS. Die resultierenden Klone wurden als mpAR4.KH 1,2,3,4 bezeichnet.

### 7.5 Einzelstrang-DNA des Klons mpAR4.KH1,2,3,4 wurde mit dem Oligonukletid KH5 mutagenisiert.

KH5: 5' AGCAAGTTGCCTGTCCTGCAATAA 3'
Die mutierten Klone wurden mittels Plaque-Hybridierung mit der Probe KH5 identifiziert. Hybridisiert wurde bei 37^{o}C in 20 % FA und 5xSSC für 16 h, gewaschen wurde bei 60^{o}C in 5xSSC/0,1 % SDS. Die resultierenden Klone wurden als mpAR4.KH1,2,3,4,5 bezeichnet.

Die ECORI Inserts der in Beispiel 6.1-6.5 beschriebenen Klone wurden wie in Beispiel 3 beschrieben in einem geeigneten eukaryontischen Expressionsvektor umkloniert und nach Transfektion (Beispiel 4) in den entsprechenden Zellen exprimiert. Die Reinigung des Proteins aus dem Zellkulturübersand erfolgt nach Standardmethoden.

## Patentansprüche

1. Reine glykosylierte, partiell glykosylierte oder nicht glykosylierte Polypeptide mit folgener Aminosäuresequenz:
1 VIGGDECNIN EHRFLVAVYE GTX¹WTFICGG VLIHPEWVIT AEHCARRRMN
51 LVFGMHRKSE KFDDEQERYP KKRYFIRCX²K TRTSWDEDIM LIRLNKPVX³N
101 SEHIAPLSLP SNPPIVGSDC RVMGWGSINR RIHVLSDEPR CANINLHX⁴FT
151 MCHGLFRKMP KKGRVLCAGD LRGRRDSCNS DSGGPLICNE ELHGIVARGP
201 NPCAQPNKPA LYTSVYDYRD WVNNVIAGX⁵A TCSP,
worin X¹, X², X³, X⁴ und X⁵ Reste natürlicher α-Aminosäuren darstellen.

2. DNA-Sequenzen, dadurch gekennzeichnet, daß sie für die Polypeptide gemäß Anspruch 1 kodieren.

3. Rekombinantes DNA-Molekül, welches eine DNA-Sequenz nach Anspruch 2 enthält.

4. Rekombinantes DNA-Molekül nach Anspruch 3, das eine DNA-Sequenz nach Anspruch 2 enthält, welche funktionell mit einer Expressions-Kontrollsequenz verbunden ist, die die Expression in geeigneten Wirtssystemen ermöglicht.

5. Rekombinantes DNA-Molekül nach Anspruch 4, dadurch gekennzeichnet, daß die Expressions-Kontrollsequenz ein in E.coli wirksames Promotersystem, ein Promotersystem eines E.coli Bakteriophagen, eine Hefe Expressions-Kontrollsequenz oder eine andere eukaryontische Expressions-Kontrollsequenz ist.

6. Wirtszelle, dadurch gekennzeichnet, daß sie mindestens ein rekombinantes DNA-Molekül gemäß Anspruch 3, 4 oder 5 enthält.

7. Wirtszelle nach Anspruch 6, dadurch gekennzeichnet, daß sie ein Bakterium, ein Pilz, eine tierische oder eine menschliche Zelle ist.

8. Gentechnisches Verfahren zur Herstellung der Polypeptide gemäß Anspruch 1, dadurch gekennzeichnet, daß man in einer geeigneten Wirtszelle DNA-Sequenzen zur Expression bringt, die für Peptidsequenzen nach Anspruch 1 kodieren.

9. Polypeptide gemäß Anspruch 1 zur Verwendung bei der Vorbeugung und Bekämpfung von Krankheiten.

10. Verwendung eines Polypeptids gemäß Anspruch 1 zur Herstellung von Arzneimitteln zur Behandlung und Prophylaxe thromboembolischer Erkrankungen und Glomerulonephritis.

## Claims

1. Pure glycosylated, partially glycosylated or unglycosylated polypeptides having the following amino-acid sequence:
1 VIGGDECNIN EHRFLVAVYE GTX¹WTFICGG VLIHPEWVIT AEHCARRRMN
51 LVFGMHRKSE KFDDEQERYP KKRYFIRCX²K TRTSWDEDIM LIRLNKPVX³N
101 SEHIAPLSLP SNPPIVGSDC RVMGWSINR RIHVLSDEPR CANINLHX⁴FT
151 MCHGLFRKMP KKGRVLCAGD LRGRRDSCNS DSGGPLICNE ELHGIVARGP
201 NPCAQPNKPA LYTSVYDYRD WVNNVIAGX⁵A TCSP,
where X¹, X², X³, X⁴ and X⁵ are residues of natural α-amino acids.

2. DNA sequences which code for the polypeptides as claimed in claim 1.

3. A recombinant DNA molecule which contains a DNA sequence as claimed in claim 2.

4. A recombinant DNA molecule as claimed in claim 3, which contains a DNA sequence as claimed in claim 2, which is functionally connected to an expression control sequence which makes expression possible in suitable host systems.

5. A recombinant DNA molecule as claimed in claim 4, wherein the expression control sequence is a promoter system effective in E.coli, a promoter system of an E.coli bacteriophage, a yeast expression control sequence or another eukaryotic expression control sequence.

6. A host cell which contains at least one recombinant DNA molecule as claimed in claim 3, 4 or 5.

7. A host cell as claimed in claim 6, which is a bacterium, a fungus, an animal or a human cell.

8. A process of genetic manipulation for the preparation of the polypeptides as claimed in claim 1, which comprises bringing about expression in a suitable host cell of DNA sequences which code for peptide sequences as claimed in claim 1.

9. Polypeptides as claimed in claim 1 for use for the prevention and control of diseases.

10. The use of a polypeptide as claimed in claim 1 for the preparation of drugs for the treatment and prophylaxis of thromboembolic disorders and glomerulonephritis.

## Revendications

1. Polypeptides absolument glycosylés, partiellement glycosylés ou non glycosylés, à la séquence d'amino-acides suivante
1 VIGGDECNIN EHRFLVAVYE GTX¹WTFICGG VLIHPEWVIT AEHCARRRMN
51 LVFGMHRKSE KFDDEQERYP KKRYFIRCX²K TRTSWDEDIM LIRLNKPVX³N
101 SEHIAPLSLP SNPPIVGSDC RVMGWGSINR RIHVLSDEPR CANINLHX⁴FT
151 MCHGLFRKMP KKGRVLCAGD LRGRRDSCNS DSGGPLICNE ELHGIVARGP
201 NPCAQPNKPA LYTSVYDYRD WVNNVIAGX⁵A TCSP,
où X¹, X², X³, X⁴ et X⁵ représentent des restes d'α-aminoacides naturels.

2. Séquences d'ADN, caractérisées par le fait quelles codent pour les polypeptides selon la revendication 1.

3. Molécule d'ADN recombinante, qui contient une séquence d'ADN selon la revendication 2.

4. Molécule d'ADN selon la revendication 3, qui contient une séquence d'ADN selon la revendication 2, qui est liée fonctionnellement à une séquence de contrôle d'expression qui permet l'expression en système hôte approprié.

5. Molécule d'ADN recombinante selon la revendication 4, caractérisée par le fait que la séquence de contrôle d'expression est un système promoteur actif dans E.Coli, un système promoteur d'un bactériophage E.Coli, une séquence de contrôle d'expression Hefe ou une autre séquence de contrôle eucaryontique.

6. Cellule hôte, caractérisée par le fait qu'elle contient au moins une molécule d'ADN recombinante selon les revendications 3, 4 ou 5.

7. Cellule hôte selon la revendication 6, caractérisée par le fait qu'elle est une bactérie, un champignon, une cellule animale ou humaine.

8. Procédé technogénique de préparation des polypeptides selon la revendication 1, caractérisé par le fait que l'on porte à l'expression, dans une cellule hôte appropriée, des séquences d'ADN qui codent pour des séquences de peptides selon la revendication 1.

9. Polypeptides selon la revendication 1 pour utilisation pour la prévention et la lutte contre les maladies.

10. Utilisation d'une polypeptide selon la revendication 1 pour la préparation de médicaments pour le traitement et la prophylaxie des maladies thromboembolitiques et des glomerulonephrites.
